# EUROPÄISCHE PATENTANMELDUNG

(11) **EP 2 338 472 A2**
(43) Veröffentlichungstag der Anmeldung: **29.06.2011**
(21) Anmeldenummer: 10177285.3
(22) Anmeldetag: 17.09.2010
(51) Int. Cl.: A61K 8/92, A61K 8/97, A61Q 5/08, A61Q 5/10, A61Q 5/12

(54) **Pflegende Haarfarbe**

(30) Priorität: 20.11.2009 DE 102009046917
(71) Anmelder: Henkel AG & Co. KGaA, 40589 Düsseldorf (DE)
(72) Erfinder: Schwartz, Stephan, 22880, Wedel (DE); Ofschanka, Britta, 22457, Hamburg (DE); Kleen, Astrid, 20457, Hamburg (DE); Akram, Mustafa, 22455, Hamburg (DE)

(57) **Zusammenfassung**

Die Erfindung betrifft ein Farbveränderungsmittel für keratinhaltige Fasern für glänzende und pflegende Färbungen. Dieses Mittel enthält in einem kosmetischen Träger neben einer farbverändernden Komponente zusätzlich eine Wirkstoffkombination aus
a. mindestens einem wässrig-alkoholischen Extrakt aus *Punica granatum* L.,
b. mindestens einem Fettsäuretriglycerid, welches einen durchschnittlichen Anteil an Estern von ungesättigten Fettsäuren von wenigstens 80 %, bezogen auf die Gesamtmenge an Fettsäureestern des Triglycerids, aufweist,
c. und mindestens ein ätherisches Öl, enthaltend einen Anteil an Terpenen von mindestens 95 Gew.-%, bezogen auf das Gesamtgewicht des pflanzlichen Öls, enthält.

Darüber hinaus betrifft die Erfindung die Verwendung des besagten Mittels zur Verbesserung des Pflegezustands, insbesondere von Glanz und Naßkämmbarkeit, und der inneren Struktur von keratinischen Fasern, insbesondere menschlichen Haaren, bei der oxidativen Färbung.

## Beschreibung

Die Erfindung betrifft ein Farbveränderungsmittel für keratinhaltige Fasern, insbesondere menschliche Haare. Dieses Mittel enthält in einem kosmetischen Träger neben einer farbverändernden Komponente eine Wirkstoffkombination aus einem wässrig-alkoholischen Extrakt aus *Punica granatum* L. sowie zwei unterschiedlichen Ölen, von welchen ein Öl als Fettsäuretriglycerid mit einem hohen Anteil an ungesättigten Fettsäuren und ein weiteres Öl durch einen hohen Anteil an Terpenen gekennzeichnet ist. Besonders vorteilhaft erweist sich der Zusatz dieser Wirkstoffkombination in oxidativen Färbemitteln. Darüber hinaus betrifft die Erfindung die Verwendung des besagten Mittels zur Verbesserung der inneren Struktur von keratinischen Fasern, insbesondere menschlichen Haaren, bei der Färbung.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Zur Bereitstellung farbverändernder kosmetischer Mittel, insbesondere für die Haut oder keratinhaltige Fasern, wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Für temporäre Färbungen werden üblicherweise Färbe- oder Tönungsmittel verwendet, die als färbende Komponente sogenannte direktziehende Farbstoffe enthalten, welche keinen oxidativen Prozess zur Ausbildung der Farbe benötigen. Eine weitere Möglichkeit zur Farbveränderung bietet die Verwendung von Färbemitteln, die sogenannte Oxofarbstoffvorprodukte, bei denen Verbindungen mit mindestens einer reaktiven Carbonylgruppe mit C,H-aciden Verbindungen und/oder Verbindungen mit primärer oder sekundärer Aminogruppe oder Hydroxygruppe, vorzugsweise aromatischen Verbindungen, in Kontakt gebracht werden und die dann in einem nichtoxidativen Prozess, der sogenannten Oxofärbung, Farbstoffe ausbilden. Die resultierenden Färbungen besitzen teilweise Farbechtheiten auf der keratinhaltigen Faser, die mit denen der Oxidationsfärbung vergleichbar sind, ohne jedoch auf Oxidationsmittel zur Farbstoffausbildung angewiesen zu sein. Bei einem weiteren Färbeverfahren werden Vorstufen des natürlichen Haarfarbstoffes Melanin auf das Haar aufgebracht, die dann im Rahmen oxidativer Prozesse im Haar naturanaloge Farbstoffe aus bilden. Die Ausfärbung kann dabei mit Luftsauerstoff als einzigem Oxidationsmittel erfolgen, so dass auf keine weiteren Oxidationsmittel zurückgegriffen werden muss.

Insbesondere oxidative Haarfärbemittel sind trotz ihrer vorteilhaften Färbeeigenschaften für den Anwender mit Nachteilen behaftet. Erstens führt der Einsatz der Oxidationsmittel zur Ausfärbung beziehungsweise Entwicklung der eigentlichen Färbung zu Schädigungen in der inneren Haarstruktur und auf der Haaroberfläche. Das Haar wird brüchig, seine Elastizität lässt nach und die Kämmbarkeit nimmt ab. Zweitens benötigen oxidative Färbemittel in der Regel einen basischen pH-Wert zur Ausfärbung, insbesondere zwischen pH 9,0 und pH 11,5. Das basische Milieu stellt einen weiteren Grund der Schädigung für das Haar und dessen innerer Struktur dar, der ebenfalls mit gesteigerter Anwendungszeit an Bedeutung gewinnt. So besteht für den Verbraucher eine gesteigerte Notwendigkeit, zusätzliche Nachbehandlungsmittel einsetzen.

Um den Aufwand der üblichen mehrstufigen Verfahren, insbesondere bei der direkten Anwendung durch Verbraucher, zu verringern, wurden in jüngster Zeit sogenannte Kombinationspräparate entwickelt. Diese Präparate enthalten neben den üblichen Komponenten, beispielsweise zur Färbung der Haare, zusätzliche Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Um den zusätzlichen Nachbehandlungsschritt einzusparen, hat es nicht daher an Versuchen gemangelt, geeignete Pflegestoffe in die Haarfärbemittel einzuarbeiten. Üblicherweise werden oxidative Haarfärbemittel unmittelbar vor der Anwendung aus einer Färbezubereitung und einer sogenannten Entwicklerzubereitung hergestellt. In der Regel besitzt die Färbezubereitung einen stark basischen pH-Wert, um die darin enthaltenen Oxidationsfarbstoffvorprodukte zu stabilisieren, während die Entwicklerzubereitung einen schwach sauren pH-Wert, dafür aber die zur Farbstoffbildung notwendigen Oxidationsmittel enthält. Beide Zubereitungen stellen somit kein vorteilhaftes Umfeld dar, einen chemisch empfindlichen Pflegestoff ohne Zersetzung zu beinhalten. Es besteht daher weiterhin ein Bedarf an geeigneten, stabilen Pflegestoffen, die sich in oxidative Farbveränderungsmittel einarbeiten lassen und so bereits während des Färbevorgangs auftretende Schädigungen zu minimieren vermögen.

Aufgabe der vorliegenden Erfindung ist es daher, die oben genannten Nachteile, insbesondere oxidativer, Haarfarbveränderungsmittel herabzusenken. Die Mittel sollen das Haar schützen und damit eine verringerte Schädigung des Haares bewirken. Insbesondere soll die innere Struktur in der Faser verbessert werden, was dem Haar erhöhte Elastizität und Geschmeidigkeit sowie einen verbesserten Glanz verleiht. Die Verringerung von Haarschädigungen während der Färbung soll jedoch nicht zu Lasten einer verringerten Färbeleistung der Mittel erreicht werden.

Es wurde nun in nicht vorhersehbarer Weise gefunden, dass der Zusatz von einer Wirkstoffkombination, die Granatapfelextrakt, ein spezielles fettes, überwiegend ungesättigtes Öl sowie ein spezifisches ätherisches Öl umfasst, in Farbveränderungsmitteln für keratinische Fasern zu Vorteilen gegenüber herkömmlichen Mitteln hinsichtlich Reduktion der inneren Faserschädigung führt.

Ein erster Gegenstand der Erfindung ist daher ein Mittel zur Färbung und/oder Aufhellung keratinischer Fasern, insbesondere menschlicher Haare, enthaltend in einem kosmetischen Träger mindestens eine farbverändernde Komponente, dadurch gekennzeichnet, dass das Mittel zusätzlich eine Wirkstoffkombination aus
(a) mindestens einem wässrig-alkoholischen Extrakt aus *Punica granatum L.,*
(b) mindestens einem Fettsäuretriglycerid, welches einen durchschnittlichen Anteil an Estern von ungesättigten Fettsäuren von wenigstens 80 %, bezogen auf die Gesamtmenge an Fettsäureestern des Triglycerids, aufweist,
(c) und mindestens ein pflanzliches Öl, enthaltend einen Anteil an Terpenen von mindestens 95 Gew.-%, bezogen auf das Gesamtgewicht des pflanzlichen Öls, enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die zur erfindungsgemäßen Verwendung eingesetzten Zubereitungen enthalten die Wirkstoffe in einem kosmetischen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Als wesentlichen Inhaltsstoff enthält das erfindungsgemäße Mittel des ersten Erfindungsgegenstands eine Wirkstoffkombination aus
mindestens einem wässrig-alkoholischen Extrakt (a) aus *Punica granatum L.,*
mindestens einem Fettsäuretriglycerid (b), welches einen durchschnittlichen Anteil an Estern von ungesättigten Fettsäuren von wenigstens 80 %, bezogen auf die Gesamtmenge an Fettsäureestern des Triglycerids, aufweist, und
mindestens einem pflanzliches Öl (c), enthaltend einen Anteil an Terpenen von mindestens 95 Gew.-%, bezogen auf das Gesamtgewicht des pflanzlichen Öls.

Als wässrig-alkoholischen Extrakt (a) aus *Punica granatum* L. (Granatapfel) wird bevorzugt ein Extrakt aus dem Fruchtfleisch des Granatapfels verwendet. Darin sind als wirksame Inhaltsstoffe insbesondere Flavonoide, Polyphenole und Phenolsäuren enthalten. Als Extraktionsmittel wird ein Gemisch aus Wasser und wasserlöslichen Alkoholen, wie Ethanol, Isopropanol, 4-Methoxybutanol, Ethyldiglycol, 1,2-Propylenglycol, 1,3-Propandiol, n-Propanol, n-Butanol, n-Butylenglycol, Glycerin, Diethylenglycolmonoethylether und Diethylenglycolmono-n-butylether enthalten. Besonders bevorzugt ist dabei ein Gemisch aus Propylenglycol und Wasser. Ein Beispiel eines solchen Extrakts trägt die INCI-Bezeichnung: Propylene Glycol, Aqua (and) Punica Granatum Fruit Extract und wird beispielsweise unter dem Handelsnamen Furitliquid Pomegranate NP von der Firma Crodarom vertrieben. Die erfindungsgemäßen Mittel enthalten bevorzugt 0,001 bis 10 Gew.-%, bevorzugt 0,01 bis 5 Gew.-% des Extrakts aus *Punica granatum* L., bezogen auf das Gesamtgewicht des anwendungsbereiten Mittels.

Als Fettsäuretriglycerid (b), welches einen durchschnittlichen Anteil an Estern von ungesättigten Fettsäuren von wenigstens 80 %, bezogen auf die Gesamtmenge an Fettsäureestern des Triglycerids, aufweist, wird erfindungsgemäß ein Triester aus Glycerin mit drei unterschiedlichen oder identischen Fettsäuren verstanden. Als Fettsäure gilt erfindungsgemäß eine aliphatische Carbonsäure mit langer, nahezu ausschließlich unverzweigter Kohlenstoff-Kette und einer Kettenlänge von 8 bis 30 Kohlenstoffatomen. Die Fettsäuren der Fettsäuretriglyceride können gesättigte oder ungesättigte Kohlenstoffketten tragen, wobei zumindest 80 Gew.-% der im Triglycerid enthaltenen Fettsäuren zumindest einfach ungesättigt sein muss und daher mindestens eine C-C-Doppelbindung aufweisen muss.

Ungesättigte Fettsäuren können eine Doppelbindung in ihrer Kohlenstoffkette sowohl in *(E)*- als auch in *(Z)*-Konfiguration besitzen, wobei bei mehreren Doppelbindungen in der Fettsäure sowohl Fettsäuren mit Doppelbindungen ausschließlich in *(Z)*-Konfiguration, ausschließlich in *(E)*-Konfiguration oder auch in gemischter Konfiguration einsetzbar sind. Bevorzugt sind jedoch ungesättigte Fettsäuren mit all-(Z)-Konfiguration. Erfindungsgemäß bevorzugte ungesättigte Fettsäuren sind Palmitoleinsäure (C16:1; 9Z), Ölsäure (C18:1; 9Z), Elaidinsäure (C18:1; 9E), Eicosensäure (Gondosäure; C20:1; 11Z), Erucasäure(C22:1; 13Z), Nervonsäure (C24:1; 15Z), Linolsäure (C18:2; 9Z, 12Z), γ-(gamma)-Linolensäure (C18:3; 6Z, 9Z, 12Z), α-(alpha)-Linolensäure (C18:3; 9Z, 12Z, 15Z), α-Elaeostearinsäure (C18:3; 9Z, 11E, 13E) und Arachidonsäure (C20:4; 5Z, 8Z, 11Z, 14Z).

Die erfindungsgemäß einsetzbaren Fettsäuretriglyceride oder Fettsäuretriglyceridgemische können an Glycerin jedoch neben Estern mit ungesättigten Fettsäuren auch anteilig Ester mit gesättigten Fettsäuren enthalten. Erfindungsgemäß bevorzugt enthält das Mittel Gemische von Fettsäuretriglyceriden, wobei Glycerin mit unterschiedlichen ungesättigten und gesättigten Fettsäuren verestert ist, solange sichergestellt ist, dass der molare Anteil an ungesättigten Fettsäuren mindestens 80 Gew.-%, bevorzugt mindestens 82 Gew.-% und insbesondere bevorzugt mindestens 85 Gew.-%, jeweils bezogen auf das Gesamtgewicht aller Fettsäuren des Fettsäuretriglycerids oder Fettsäuretriglyceridgemischs, beträgt.

Erfindungsgemäß bevorzugt einsetzbare Fettsäuretriglyceride sind dabei ein Gemisch von Fettsäuretriglyceriden, das sich dadurch auszeichnet, dass der Anteil an Linolsäure (C18:2; 9Z, 12Z; auch (*all*-Z)-9,12,-Octadiensäure) unter den Fettsäuren mindestens 20 Gew.-%, bevorzugt mindestens 22 Gew.-% und insbesondere mindestens 25 Gew.-%, jeweils bezogen auf das Gesamtgewicht aller Fettsäuren des Fettsäuretriglycerids oder Fettsäuretriglyceridgemischs, beträgt. Eine Ausführungsform des ersten Erfindungsgegenstands ist daher dadurch gekennzeichnet, dass das Fettsäuretriglycerid (b) einen durchschnittlichen Anteil an Estern von Linolsäure von wenigstens 25 %, bezogen auf die Gesamtmenge an Fettsäureestern des Triglycerids, aufweist.

Erfindungsgemäße Mittel enthalten als Fettsäuretriglyceride oder Fettsäuretriglyceridgemisch mit einem hohen Anteil an ungesättigten Fettsäuren. Ein Maß für die Ungesättigtheit einer Verbindung bzw. eines Gemisches von Verbindungen stellt die sogenannte Iodzahl dar. Damit lässt sich, insbesondere bei Gemischen, der Anteil an C-C-Doppelbindungen bestimmen, wobei eine hohe Zahl für einen hohen Anteil an Doppelbindungen steht. Dem Fachmann ist die Bestimmung der lod- Zahl wohl bekannt, sie kann beispielsweise nach der Methode DGF C-V-11 bestimmt werden. Erfindungsgemäß bevorzugt sind dabei insbesondere Fettsäuretriglyceride oder Fettsäuretriglyceridgemische, welche eine lodzahl von mindestens 95, bevorzugt von mindestens 100 und insbesondere von 100 bis 120 besitzen.

Als erfindungsgemäße Fettsäuretriglyceride oder Fettsäuretriglyceridgemische eignen sich insbesondere natürliche Öle, welche die vorgenannten Merkmale besitzen und welche vorzugsweise pflanzlicher Herkunft sind. Als erfindungsgemäß besonders geeignet hat sich ein Öl aus den Samen/Kernen von Steinfrüchten, insbesondere von *Prunus Armeniaca* (Aprikose) herausgestellt. Aprikosenkernöl wird entweder durch mechanische Pressung aus den Samen von *Prunus Armeniaca* und anschließender Raffination als fettes Öl oder durch Extraktion und anschließende Raffination erhalten, wobei durch Pressung, insbesondere durch kalte Pressung, gewonnenes Öl bevorzugt ist.

Eine weitere Ausführungsform des ersten Erfindungsgegenstands ist daher dadurch gekennzeichnet, dass das Mittel als Fettsäuretriglycerid (b) das Kernöl von *Prunus Armeniaca* (Aprikosenkernöl) enthält.

Erfindungsgemäß bevorzugte Mittel sind dadurch gekennzeichnet, dass das Mittel das Fettsäuretriglycerid oder Fettsäuretriglyceridgemisch mit einen Anteil an ungesättigten Fettsäuren von mindestens 80 Gew.-%, bezogen auf das Gesamtgewicht aller Fettsäuren des Fettsäuretriglycerids oder Fettsäuretriglyceridgemischs, in einem Gewichtsanteil von 0,001 bis 5 Gew.-%, vorzugsweise von 0,01 bis 3 Gew.-% und insbesondere von 0,1 bis 1,5 Gew.-%, jeweils bezogen auf das Gesamtgewicht des anwendungsbereiten Mittels, enthält.

Unter einem ätherischen Öl (c), enthaltend einen Anteil an Terpenen von mindestens 95 Gew.-%, bezogen auf das Gesamtgewicht des ätherischen Öls, wird erfindungsgemäß ein Öl verstanden, welches sich überwiegend aus flüchtigen, nicht triglyceridischen Inhaltsstoffen zusammensetzt.

Flüchtige, nicht triglyceridsche Inhaltsstoffe der ätherischen Öle sind erfindungsgemäß im wesentlich Terpene und terpenoide Verbindungen, insbesondere acyclische und cyclische Monoterpene. Dazu zählen insbesondere die Verbindungen Citral, Pinen, Linalool, Geraniol, Geranial, Nerol und Limonen. Bevorzugte erfindungsgemäße Mittel enthalten ein ätherisches Öl, welches überwiegend aus Limonen, bevorzugt zu mindestens 90 Gew.-%, und besonders bevorzugt zu mindestens 95 Gew.-%, jeweils bezogen auf das Gesamtgewicht des ätherischen Öls, aus Limonen besteht.

Eine weitere Ausführungsform des ersten Erfindungsgegenstands ist daher dadurch gekennzeichnet, dass das Mittel ein ätherisches Öl (c) enthält, welches einen durchschnittlichen Anteil an Limonen von wenigstens 90 Gew.-%, bezogen auf das Gesamtgewicht des ätherischen Öls (c), aufweist.

Geeignete ätherische Öle sind dabei insbesondere Öle, welche aus den Schalen von Citrusfrüchten, z. B. durch mechanische Pressung, gewonnen werden. Zu geeigneten Citrusfrüchten zählen Süßorange *(Citrus sinensis),* Bitterorange, Grapefruit und Mandarine. Bevorzugt sind dabei ätherische Öle aus den Schalen der Süßorange.

Eine weitere Ausführungsform des ersten Erfindungsgegenstands ist daher dadurch gekennzeichnet, dass das Mittel als ätherisches Öl (c) ein Öl aus Schalen von *Citrus sinensis* enthält.

Das erfindungsgemäße Mittel enthält das ätherische Öl (c) bevorzugt in einem Gewichtsanteil von 0,001 bis 2 Gew.-%, bevorzugt von 0,01 bis 1,5 Gew.-%, und insbesondere von 0,05 bis 1,0 Gew.-% enthält, wobei sich die Gewichtsanteile jeweils auf das Gesamtgewicht des anwendungsbereiten Mittels beziehen.

Besonders bevorzugte Mittel sind dadurch gekennzeichnet, dass sie als Wirkstoffkombination mindestens einem wässrig-alkoholischen Extrakt aus *Punica granatum* L. mit mindestens Aprikosenkernöl als Fettsäuretriglycerid (b) und mindestens ein Öl aus den Schalen der Süßorange als ätherisches Öl (c) enthalten.

Eine weitere Ausführungsform des ersten Erfindungsgegenstands ist dabei dadurch gekennzeichnet, dass das Mittel das Fettsäuretriglycerid (b) in einem Gewichtsanteil von 0,001 bis 5 Gew.-%, bevorzugt von 0,01 bis 3 Gew.-%, und insbesondere von 0,1 bis 1,5 Gew.-% und das ätherische Öl (c) in einem Gewichtsanteil von 0,001 bis 2 Gew.-%, bevorzugt von 0,01 bis 1,5 Gew.-%, und insbesondere von 0,05 bis 1,0 Gew.-% enthält, wobei sich die Gewichtsanteile jeweils auf das Gesamtgewicht des anwendungsbereiten Mittels beziehen.

Als weiteren wesentlichen Inhaltsstoff enthält die erfindungsgemäße Färbezubereitung mindestens eine farbverändernde und/oder farbgebende Komponente. Die farbverändernde Komponente wird dabei ausgewählt aus
(a) mindestens einem Oxidationsfarbstoffvorprodukt und/oder
(b) mindestens einem direktziehenden Farbstoff und/oder
(c) mindestens einem naturanalogen Farbstoff und/oder
(d) mindestens einem Oxofarbstoffvorprodukt und/oder
(e) mindestens einem Aufhellmittel in Form eines chemischen Oxidationsmittels.

In einer Ausführungsform des ersten Erfindungsgegenstands ist das erfindungsgemäße Mittel dadurch gekennzeichnet, dass die farbverändernde Komponente ausgewählt ist aus mindestens einem Oxidationsfarbstoffvorprodukt vom Typ der Entwicklerkomponenten und gegebenenfalls zusätzlich mindestens einer Kupplerkomponente und/oder aus mindestens einem direktziehenden Farbstoff.

In einer bevorzugten Ausführungsform des ersten Erfindungsgegenstands enthält das Mittel als farbgebende Komponente mindestens ein Oxidationsfarbstoffvorprodukt. Als Oxidationsfarbstoffvorprodukte enthalten die Zubereitungen mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Mittel daher mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetra-oxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, beispielsweise aus Pyrimidin-Derivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Als Pyrazolopyrimidine sind insbesondere Pyrazolo-[1,5-a]pyrimidine bevorzugt, wobei bevorzugte Pyrazolo[1,5-a]pyrimidine ausgewählt sind aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]-pyrimidin-7-ol, 3-Aminopyrazolo[1,5-a]pyrimidin-5-ol, 2-(3-Aminopyrazolo[1,5-a]pyrimidin-7-yl-amino)ethanol, 2-(7-Aminopyrazolo[1,5-a]pyrimidin-3-ylamino)ethanol, 2-[(3-Aminopyrazolo[1,5-a]-pyrimidin-7-yl)-(2-hydroxyethyl)amino]ethanol, 2-[(7-Aminopyrazolo[1,5-a]pyrimidin-3-yl)-(2-hydroxy ethyl)amino]ethanol, 5,6-Dimethylpyrazolo[1,5-a]pyrimidin-3,7-diamin, 2,6-Dimethylpyrazolo[1,5-a]-pyrimidin-3,7-diamin, 3-Amino-7-dimethylamino-2,5-dimethylpyrazolo[1,5-a]pyrimidin sowie ihre physiologisch verträglichen Salze und ihre tautomeren Formen.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie deren physiologisch verträglichen Salzen. Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze. Die Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Erfindungsgemäße Kupplerkomponenten werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidin-derivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetra-hydrochinoxalin, und/oder Gemischen aus zwei oder mehreren Verbindungen aus einer oder mehreren dieser Klassen.

Die erfindungsgemäß verwendbaren m-Aminophenole bzw. deren Derivate werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Die erfindungsgemäß verwendbaren 3-Diaminobenzole bzw. deren Derivate werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Die erfindungsgemäß verwendbaren o-Diaminobenzole bzw. deren Derivate werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Die erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Die erfindungsgemäß verwendbaren Indolderivate werden bevorzugt ausgewählt aus 4-Hydroxyindol, 6-Hydroxyindol und 7-Hydroxyindol und deren physiologisch verträglichen Salzen. Die erfindungsgemäß verwendbaren Indolinderivate werden bevorzugt ausgewählt aus 4-Hydroxyindolin, 6-Hydroxyindolin und 7-Hydroxyindolin und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)-propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)-ethanol, 2-({3-[(2-Hydroxyethyl)amino]-4,5-dimethylphenyl}amino)ethanol, 2-[3-Morpholin-4-yl-phenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Oxidationsfarbstoffvorprodukte vom Entwicklertyp und vom Kupplertyp werden besonders bevorzugt in bestimmten Kombinationen eingesetzt. Mit den als Kombination genannten Oxidationsfarbstoffvorprodukten und/oder deren physiologisch verträglichen Salzen können jedoch auch noch weitere Farbstoffvorprodukte kombiniert werden: p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin;p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis-(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)-ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1H-imidazol-1-yl)propyl]amin / 5-Amino-2-methylphenol; N-(4-Amino-3-methylphenyl)-N-[3-(1H-imidazol-1-yl)propyl]amin / 3-Aminophenol; N-(4-Amino-3-methylphenyl)-N-[3-(1H-imidazol-1-yl)-propyl]amin / 2-(2,4-Diaminophenoxy)ethanol; N-(4-Amino-3-methylphenyl)-N-[3-(1H-imidazol-1-yl)-propyl]amin / 1,3-Bis(2,4-diaminophenoxy)propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)propan; 4,5-Diamino-1-(2-hydroxyethyl)pyrazol / 1-Methoxy-2-amino-4-(2-hydroxyethylamino)benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

Um eine ausgewogene und subtile Nuancenausbildung zu erzielen, ist es erfindungsgemäß vorteilhaft, wenn die erfindungsgemäßen Mittel weitere farbgebende Komponenten enthalten.

Weiterhin können die Mittel zur erfindungsgemäßen Verwendung mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Als anionische direktziehende Farbstoffe eignen sich insbesondere FD&C Yellow No. 6 (C.1. 15,985), Acid Yellow 1 (C.I. 10,316), Acid Yellow 3 (C.I. 47,005), Acid Yellow 9 (C.I. 13,015), Acid Yellow 23 (C.I. 19,140), Acid Yellow 36 (C.I. 13,065), Acid Yellow 73 (C.I. 45,350), Acid Orange 3 (C.I. 10,385), Acid Orange 6 (C.I. 14,270), Acid Orange 7 (C.I. 15,510), Acid Orange 24 (C.I. 20,170), Acid Red 4 (C.I. 14,710), Acid Red 14 (C.I. 14,720), Acid Red 18 (C.I. 16,255), Acid Red 27 (C.I. 16,185), Acid Red 33 (C.I. 17,200), Acid Red 35 (C.I. 18,065), Acid Red 51 (C.I. 45,430), Acid Red 52 (C.I. 45,100), Acid Red 73 (C.I. 27,290), Acid Red 87 (C.I. 45,380), Acid Red 92 (C.I. 45,410), Acid Red 95 (C.I. 45425), Acid Red 184 (C.I. 15,685), Acid Red 195, Pigment Red 57:1 (C.I. 15,850:1), FD&C Red No. 4 (C.I. 14,700), Acid Green 25 (C.I. 61,570), Acid Green 50 (C.I. 44,090), Acid Blue 1 (C.I. 42,045), Acid Blue 3 (C.I. 42,051 Acid Blue 7 (C.I. 42,080), Acid Blue 9 (C.I. 42,090), Acid Blue 25 (C.I. 62,055), Acid Blue 62 (C.I. 62045), Acid Blue 74 (C.I. 73,015), Acid Violet 9 (C.I. 45,190), Acid Violet 43 (C.I. 60,730), Acid Brown 13 (C.I. 10,410), Acid Black 1 (C.I. 20,470), Acid Black 52 (C.I. 15,711), Food Black No. 1 (C.I. 28,440), 3',3",5',5"-Tetrabromphenolsulfonphthalein (Bromphenolblau), 3,3',3",4,5,5',5",6-Octabromphenolsulfonphthalein (Tetrabromphenolblau). Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen.

Als kationische direktziehende Farbstoffe eignen sich insbesondere Basic Blue 6 (C.I. 51,175), Basic Blue 7 (C.I. 42,595), Basic Blue 8 (C.I. 42,563), Basic Blue 9 (C.I. 52,015), Basic Blue 26 (C.I. 44,045), Basic Blue 41 (C.I. 11,154), Basic Blue 99 (C.I. 56,059), Basic Violet 1 (C.I. 42,535), Basic Violet 2 (C.I. 42,520), Basic Violet 3 (C.I. 42,555), Basic Violet 10 (C.I. 45,170), Basic Violet 14 (C.I. 42,510), Basic Brown 4 (C.I. 21,010), Basic Brown 16 (C.I. 12,250), 1-[(4-Amino-2-nitrophenyl)azo]-7-(trimethylammonio)-2-naphthol-chlorid, Basic Brown 17 (C.I. 12,251), Basic Orange 69 (C.I. 12,605), Basic Red 2 (C.I. 50,240), Basic Red 22 (C.I. 11,055), Basic Red 76 (C.I. 12,245), Basic Yellow 2 (C.I. 41,000), Basic Yellow 11 (C.I. 48,055), Basic Yellow 57 (C.I. 12,719), Basic Green 1 (C.I. 42,040), Basic Green 4 (C.I. 42,000), 1-(2-Morpholiniumpropylamino)-4-hydroxy-9,10-anthrachinon-methylsulfat, 1-[(3-(Dimethylpropylaminium)propyl)amino]-4-(methylamino)-9,10-anthrachinon-chlorid, HC Blue 16 (Bluequat B) und direktziehende Farbstoffe, die einen Heterocyclus enthalten, der mindestens ein quaternäres Stickstoffatom aufweist. Bevorzugte kationische direktziehende Farbstoffe sind dabei Basic Blue 7, Basic Blue 26, Basic Violet 2, Basic Violet 14, Basic Yellow 57, Basic Red 76, Basic Blue 99, Basic Brown 16, Basic Brown 17, HC Blue 16, Basic Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Geeignete Nitrofarbstoffe sind insbesondere ausgewählt aus 1,4-Bis[(2-hydroxyethyl)amino]-2-nitrobenzol, HC Blue 2, HC Blue 6, HC Blue 9, HC Blue 10, HC Blue 11, HC Blue 12, HC Blue 13, HC Violet 1, HC Violet 2, 1-(2-Aminoethylamino)-4-[di(2-hydroxyethyl)amino]-2-nitrobenzol, 4-(Di(2-hydroxyethyl)amino)-2-nitro-1-phenylamino-benzol, HC Red 7, 2-Amino-4,6-dinitrophenol, C.I. 76,070, HC Red 1, HC Red 13, 1-Amino-4-[(2-hydroxyethyl)-amino]-5-chlor-2-nitrobenzol, HC Red 3, 4-[(2-Hydroxyethyl)methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)amino]benzol, 4-Amino-3-nitrophenol, 4-[(2-Hydroxyethyl)amino]-3-nitrophenol, HC Orange 1, HC Orange 2, HC Orange 3, HC Red 10, HC Red 11, 2-[(2-Hydroxyethyl)amino]-4,6-dinitrophenol, 4-Ethylamino-3-nitrobenzoesäure, 2-[(4-Amino-2-nitrophenyl)amino]benzoesäure, 2-Chlor-6-ethylamino-4-nitrophenol, 2-Amino-6-chlor-4-nitrophenol, HC Red BN, 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3-Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, HC Red 14, C.I. 76,020, HC Yellow 2, HC Yellow 4, HC Yellow 5, HC Yellow 6, 2-[Di(2-hydroxyethyl)-amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, HC Yellow 9, HC Yellow 10, HC Yellow 11, 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, HC Yellow 12, HC Yellow 13, HC Yellow 14, HC Yellow 15, 3-[(2-Hydroxyethyl)amino]-4-methyl-1-nitrobenzol und 4-Chlor-3-[(2-hydroxyethyl)amino]-1-nitrobenzol. Geeignete Chinonfarbstoffe sind insbesondere ausgewählt aus 1,4-Di[(2,3-dihydroxypropyl)amino]-9,10-anthrachinon, Disperse Blue 23 (C.I. 61,545), Disperse Blue 3 (C.I. 61,505), HC Orange 5, Disperse Red 15 (C.I. 60,710), 1-Hydroxy-4-[(4-methyl-2-sulfophenyl)amino]-9,10-anthrachinon, Natural Red 4 (C.I. 75,470), HC Blue 8, HC Red 8, Disperse Red 11 (C.I. 62,015), Disperse Blue 7 (C.I. 62,500), Disperse Violet 1 (C.I. 61,100), Disperse Violet 4 (C.I. 61,105), 2-Hydroxy-3-methoxy-1,4-naphthochinon, 2,5-Dihydroxy-1,4-naphthochinon, 2-Hydroxy-3-methyl-1,4-naphthochinon, HC Red 9, HC Green 1, Natural Brown 7 (C.I. 75,500), Natural Orange 6 (C.I. 75,480), C.I. 73,000, 4-{{5-[(2-Hydroxyethyl) amino]-1-methyl-1H-pyrazol-4-yl}imino}-4,5-dihydro-5-[(2-hydroxyethyl)imino]-1-methyl-1H-pyrazol-sulfat(1:1), Hydrat(1:1). Geeignete neutrale Azofarbstoffe sind insbesondere ausgewählt aus Disperse Red 17 (C.I. 11,210), Disperse Black 9, HC Yellow 7, 2,6-Diamino-3-[(pyridin-3-yl)azo]pyridin, Disperse Yellow 3 (C.I. 11855), Disperse Orange 3 (C.I. 11,005).

Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Als Farbstoffvorstufen naturanaloger Farbstoffe werden bevorzugt solche substituierte Indole und Indoline eingesetzt. In einer weiteren Ausführungsform enthalten die Färbemittel daher mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Besonders geeignet als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und/oder 5,6-Dihydroxyindolin-2-carbonsäure, insbesondere bevorzugt 5,6-Dihydroxyindolin, sowie deren physiologisch verträgliche Salze. Als Vorstufen naturanaloger Haarfarbstoffe hervorragend geeignet sind weiterhin Derivate des 5,6-Dihydroxyindols , insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere bevorzugt 5,6-Dihydroxyindol, sowie deren physiologisch verträgliche Salze.

Eine weitere Möglichkeit zur Farbveränderung bietet die Verwendung von Färbemitteln, die sogenannte Oxofarbstoffvorprodukte enthalten. Eine erste Klasse der Oxofarbstoffvorprodukte sind Verbindungen mit mindestens einer reaktiven Carbonylgruppe. Diese erste Klasse wird als Komponente (Oxo1) bezeichnet. Eine zweite Klasse der Oxofarbstoffvorprodukte bilden C,H-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Diese zweite Klasse wird als Komponente (Oxo2) bezeichnet. In Kombination bilden sie in einem nichtoxidativen Prozess der sogenannten Oxofärbung Farbstoffe aus. Unter Verbindungen der Komponente (Oxo2) können allerdings auch entsprechende Oxidationsfarbstoffvorprodukte vom Entwickler- und/oder Kupplertyp mit oder ohne Einsatz eines Oxidationsmittels Verwendung finden. Somit lässt sich die Methode der Oxofärbung ohne weiteres mit dem oxidativen Färbesystem kombinieren.

In einer weiteren Ausführungsform der erfindungsgemäßen Verwendung können daher als farbverändernde Komponente daher auch Oxofarbstoffvorprodukte eingesetzt werden. Oxofarbstoffvorprodukte werden bevorzugt als Kombination aus mindestens einer Verbindung, die mindestens eine reaktive Carbonylgruppe enthält (Komponente (Oxo1)) mit mindestens einer Verbindung (Komponente Oxo2), ausgewählt aus C,H-aciden Verbindungen (Oxo2a) und/oder aus Verbindungen (Oxo2b) mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterocyclischen Verbindungen und aromatischen Hydroxyverbindungen, eingesetzt.

Reaktive Carbonylverbindungen als Komponente (Oxo1) besitzen im Sinne der Erfindung mindestens eine Carbonylgruppe als reaktive Gruppe, welche mit der Komponente (Oxo2) unter Ausbildung einer kovalenten Bindung reagiert.

Bevorzugte reaktive Carbonylverbindungen der Komponente (Oxo1) werden ausgewählt aus der Gruppe, bestehend aus Benzaldehyd, Naphthaldehyd, Zimtaldehyd und den Derivaten dieser Aldehyde, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)acetaldehyd, 1-Methylpyrrol-2-aldehyd, Pyridoxal, Antipyrin-4-aldehyd, Furfural, 5-Nitrofurfural, Chromon-3-aldehyd, 3-(5'-Nitro-2'-furyl)acrolein, 3-(2'-Furyl)acrolein und Imidazol-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)-penta-2,4-dienal, 5-(4-Pyrrolidinophenyl)penta-2,4-dienal, 5-(4-Dimethylamino-1-naphthyl)penta-3,5-dienal, Piperonal, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, Salze, deren kationische Komponente 4-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethylpyridinium, 4-Formyl-1,3-dimethylpyridinium, 4-Formyl-1-methylchinolinium, 2-Formyl-1-methylchinolinium, 5-Formyl-1-methylchinolinium, 6-Formyl-1-methylchinolinium, 7-Formyl-1-methylchinolinium, 8-Formyl-1-methylchinolinium, 5-Formyl-1-ethylchinolinium, 6-Formyl-1-ethyl-chinolinium, 7-Formyl-1-ethylchinolinium, 8-Formyl-1-ethylchinolinium, 5-Formyl-1-benzylchinolinium, 6-Formyl-1-benzylchinolinium, 7-Formyl-1-benzylchinolinium, 8-Formyl-1-benzylchinolinium, 5-Formyl-1-allylchinolinium, 6-Formyl-1-allylchinolinium, 7-Formyl-1-allylchinolinium oder 8-Formyl-1-allylchinolinium ist und deren anionisches Gegenion ausgewählt aus Benzolsulfonat, p-Toluolsulfonat, Methansulfonat, Perchlorat, Sulfat, Chlorid, Bromid, lodid, Tetrachlorozinkat, Methylsulfat, Trifluormethansulfonat oder Tetrafluoroborat ist, Isatin, 1-Methylisatin, 1-Allylisatin, 1-Hydroxymethylisatin, 5-Chlorisatin, 5-Methoxyisatin, 5-Nitroisatin, 6-Nitroisatin, 5-Sulfoisatin, 5-Carboxyisatin, Chinisatin, 1-Methylchinisatin, sowie beliebigen Gemischen der voranstehenden Verbindungen.

Als C,H-acide Verbindungen (Oxo2a) sind solche Verbindungen angesehen, die ein an ein aliphatisches Kohlenstoffatom gebundenes Wasserstoffatom tragen, wobei aufgrund von Elektronenziehenden Substituenten eine Aktivierung der entsprechenden Kohlenstoff-Wasserstoff-Bindung bewirkt wird. Bevorzugte Verbindungen (Oxo2a) werden ausgewählt aus einer oder mehrerer Verbindungen der Gruppe von Salzen mit physiologisch verträglichem Gegenion, die gebildet wird aus Salzen des 1,2-Dihydro-1,3,4,6-tetramethyl-2-oxo-pyrimidiniums, 1,2-Dihydro-1,3,4-trimethyl-2-oxo-pyrimidiniums, 1-Allyl-1,2-dihydro-3,4,6-trimethyl-2-oxo-pyrimidiniums, 1,2-Dihydro-1-(2-hydroxyethyl)-3,4,6-trimethyl-2-oxo-pyrimidiniums und 1,2-Dihydro-1,3,4,6-tetramethyl-2-thioxo-pyrimidiniums, wobei als Gegenion bevorzugt Halogenide, Benzolsulfonat, p-Toluolsulfonat, (C₁-C₄-Alkan)sulfonat, Trifluormethansulfonat, Perchlorat, 0,5 Sulfat, Hydrogensulfat, Tetrafluoroborat, Hexafluorophosphat oder Tetrachlorozinkat eingesetzt werden. Weiterhin bevorzugte Verbindungen (Oxo2a) sind ausgewählt aus 2-(2-Furoyl)acetonitril, 2-(5-Brom-2-furoyl)acetonitril, 3-(2,5-Dimethyl-3-furyl)-3-oxopropanitril, 2-(2-Thenoyl)acetonitril, 2-(3-Thenoyl)acetonitril, 2-(5-Fluor-2-thenoyl)acetonitril, 2-(5-Chlor-2-thenoyl)acetonitril, 2-(5-Brom-2-thenoyl)acetonitril, 2-(5-Methyl-2-thenoyl)acetonitril, 2-(2,5-Dimethylpyrrol-3-oyl)acetonitril, 2-(1,2,5-Trimethylpyrrol-3-oyl)acetonitril, 1*H*-Benzimidazol-2-ylacetonitril, 1*H*-Benzothiazol-2-ylacetonitril, 2-(Pyrid-2-yl)acetonitril, 2,6-Bis-(cyanomethyl)pyridin, 2-(Indol-3-oyl)acetonitril, 2-(2-Methyl-indol-3-oyl)acetonitril und 2-(6-Hydroxy-4,7-dimethoxy-1-benzofuran-5-oyl)acetonitril, insbesondere 1H-Benzimidazol-2-ylacetonitril.

Des Weiteren kann als Komponente (Oxo2b) mindestens ein Oxidationsfarbstoffvorprodukt mit mindestens einer primären oder sekundären Aminogruppe und/oder mindestens einer Hydroxygruppe verwendet werden. Bevorzugt geeignete Vertreter finden sich unter der Ausführung der Oxidationsfarbstoffvorprodukte. Es ist jedoch erfindungsgemäß bevorzugt, wenn die Verbindungen der Komponente (Oxo2) nur unter C,H-aciden Verbindungen ausgewählt werden.

Die voranstehend genannten Verbindungen der Komponente (Oxo1) sowie der Komponente (Oxo2) werden, wenn sie zum Einsatz kommen, jeweils vorzugsweise in einer Menge von 0,001 bis 10 Gew.-%, insbesondere von 0,01 bis 5 Gew.-%, jeweils bezogen auf Gesamtgewicht des anwendungsbereiten Mittels, verwendet.

In einer weiteren Ausführungsform enthält das erfindungsgemäße Mittel als farbverändernde Komponente mindestens ein Aufhellmittel in Form eines chemischen Oxidationsmittels. Bevorzugt wird ein chemisches Oxidationsmittel in Kombination mit einem farbgebenden Mittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Bevorzugte chemische Oxidationsmittel sind Peroxide, Peroxodisulfate, Chlorite, Hypochlorite und insbesondere Wasserstoffperoxid oder dessen Anlagerungsprodukte an Harnstoff, Melamin sowie Natriumborat.

Eine bevorzugte Ausführungsform der erfindungsgemäßen Verwendung ist daher dadurch gekennzeichnet, dass das Mittel zusätzlich mindestens ein Oxidationsmittel, ausgewählt aus Wasserstoffperoxid und/oder einen seiner Anlagerungsprodukte an organische oder anorganische Verbindungen, enthält. Bevorzugte Anlagerungsprodukte sind die Anlagerungsprodukte von Wasserstoffperoxid an Harnstoff, Melamin sowie Natriumborat. Bevorzugt wird jedoch als Oxidationsmittel Wasserstoffperoxid in Form einer Lösung eingesetzt. Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges H₂O₂), jeweils bezogen auf das anwendungsbereite Mittel.

Zur Steigerung der Aufhellwirkung von Wasserstoffperoxid ist es möglich, Bleichkraftverstärker zuzugeben. Hierzu zählen Persulfat-Salze, Siliciumdioxid-Verbindungen sowie insbesondere kationisierte Heterocyclen. Zur weiteren Steigerung der Leistung der Oxidationsmittelzubereitung kann der erfindungsgemäßen Zusammensetzung daher zusätzlich mindestens eine gegebenenfalls hydratisierte SiO₂-Verbindung zugesetzt werden. Es kann erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder. Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere deren Kalium- und Natriumsalze. Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder als Wasserglas eingesetzt. Erfindungsgemäß besonders bevorzugt sind Wassergläser. Geeignete Bleichkraftverstärker vom Typ kationisierter Heterocyclen sind insbesondere physiologisch verträgliche Salze von Acetyl-1-methylpyridinium, 4-Acetyl-1-methylpyridinium und N-Methyl-3,4-dihydroisochinolinium, besonders bevorzugt 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Eine oxidative Färbung der Fasern kann in Gegenwart von Oxidationsfarbstoffvorprodukten grundsätzlich mit Luftsauerstoff erfolgen. Es ist jedoch vorteilhaft, dem Färbemittel, enthaltend Oxidationsfarbstoffvorprodukte, ein chemisches Oxidationsmittel zuzusetzen, um den Farbbildungsprozess zu beschleunigen und/oder einen Aufhelleffekt zu erzielen. Besonders geeignete Oxidationsmittel sind Wasserstoffperoxid oder dessen Anlagerungsprodukte an Harnstoff, Melamin sowie Natriumborat.

Erfindungsgemäß kann das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. Enzyme, lodide, Chinone oder Metallionen.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht. Bei einer Anwendung von zusätzlichen Oxidationsmitteln wird die eigentliche Färbezubereitung zweckmäßigerweise unmittelbar vor der Anwendung durch Mischung einer erfindungsgemäßen Zubereitung, enthaltend mindestens eine farbverändernde Komponente sowie der erfindungsgemäßem Wirkstoffkombination, sowie einer Zubereitung, enthaltend das zusätzliche Oxidationsmittel, insbesondere Wasserstoffperoxid, hergestellt.

Eine weitere Ausführungsform des ersten Erfindungsgegenstands ist daher dadurch gekennzeichnet, dass das anwendungsbereite Mittel mindestens ein Oxidationsfarbstoffvorprodukt vom Typ der Entwicklerkomponenten, mindestens ein Oxidationsfarbstoffvorprodukt vom Typ der Kupplerkomponente und zusätzlich mindestens ein chemisches Oxidationsmittel, insbesondere Wasserstoffperoxid, enthält.

Weiterhin hat es sich als vorteilhaft erweisen, wenn die Farbveränderungsmittel mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Bevorzugte anionische Tenside sind Seifen, Alkylsulfate, Alkylethersulfate und Ethercarbonsäuren mit 10 bis 18 C-Atomen in Alkylgruppe und bis zu 12 Glykolethergruppen im Molekül. Ein bevorzugtes zwitterionisches Tensid ist das unter der INCl-Bezeichnung Cocamidopropyl Betaine bekannte Fettsäureamid-Derivat. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate und Bezeichnung Disodium Cocoamphodiacetate vermarktet.

Als nichtionische Tenside eignen sich insbesondere C₈-C₂₂-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie Assoziativpolymere mit Fettalkylkette, kationische Polymere, nichtionische Polymere; zwitterionische und amphotere Polymere; anionische Polymere; Verdickungsmittel; haarkonditionierende Verbindungen; Proteinhydrolysate pflanzlicher oder tierischer Herkunft; Parfümöle, Dimethylisosorbid und Cyclodextrine; faserstrukturverbessernde Wirkstoffe; Entschäumer wie Silikone; Farbstoffe zum Anfärben des Mittels; Antischuppenwirkstoffe; Lichtschutzmittel; Wirkstoffe (Pantolacton, Allantoin, Pyrrolidinoncarbonsäuren und deren Salze sowie Bisabolol); Vitamine, Provitamine und Vitaminvorstufen; Pflanzenextrakte; pflanzliche Öle; Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe; Trübungsmittel; Perlglanzmittel; Treibmittel wie Propan-Butan-Gemische, N₂O, Dimethylether, CO₂ und Luft; Antioxidantien.

Die Auswahl dieser weiteren Stoffe wird der Fachmann gemäß den gewünschten Eigenschaften der Zubereitungen treffen. Die Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die Färbezubereitungen der erfindungsgemäßen Verwendung weisen bevorzugt einen pH-Wert im Bereich von 4 bis 12 aufweist. Im Fall von Oxidationsfärbemitteln findet die Anwendung der Färbemittel in einem schwach alkalischen Milieu statt, bevorzugt bei einem pH-Wert im Bereich von 8,0 bis 10,5. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden ausgewählt aus 2-Aminoethan-1-ol (Monoethanolamin), 2-Amino-2-methylpropan-1-ol, 2-Amino-2-methyl-propan-1,3-diol und Triethanolamin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat, bevorzugt Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin. Schließlich ist ein weiteres bevorzugtes Alkalisierungsmittel Ammoniak. Bevorzugt werden Acidificierungsmittel und Alkalisierungsmittel jeweils in einer Menge von 0,05 bis 15 Gew.-%, insbesondere von 0,5 bis 10 Gew.-%, bezogen auf das Gesamtgewicht des anwendungsbereiten Mittels, eingesetzt.

Die erfindungsgemäßen Mittel können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbemittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein weiterer Gegenstand der vorliegenden Erfindung ist daher eine Mehrkomponentenverpackungseinheit (Kit-of-Parts), welche mindestens zwei voneinander getrennt konfektionierte Verpackungseinheiten (Container) umfasst und dadurch gekennzeichnet ist, dass ein Container C1 in einem kosmetischen Träger mindestens ein Oxidationsfarbstoffvorprodukt vom Typ der Entwicklerkomponenten, gegebenenfalls zusätzlich mindestens einer Kupplerkomponente, sowie mindestens eine Wirkstoffkombination aus
(a) mindestens einem wässrig-alkoholischen Extrakt aus *Punica granatum L.,*
(b) mindestens einem Fettsäuretriglycerid, welches einen durchschnittlichen Anteil an Estern von ungesättigten Fettsäuren von wenigstens 80 %, bezogen auf die Gesamtmenge an Fettsäureestern des Triglycerids, aufweist,
(c) und mindestens ein ätherisches Öl, enthaltend einen Anteil an Terpenen von
   mindestens 95 Gew.-%, bezogen auf das Gesamtgewicht des pflanzlichen Öls, enthält, und dass ein weiterer Container C2 in einem kosmetischen Träger mindestens ein chemisches Oxidationsmittel, insbesondere Wasserstoffperoxid, enthält.

In einer besonders bevorzugten Ausführungsform ist die Verpackungseinheit des zweiten Erfindungsgegenstands dadurch gekennzeichnet, dass sie mindestens eine zusätzliche Komponente, ausgewählt aus der Gruppe, die gebildet wird aus persönlicher Schutzbekleidung, wie Einweghandschuhen, Schürze, Applikationshilfe, wie Kamm, Bürste, Pinsel oder Applicette, und Gebrauchsanleitung enthält. Die Gebrauchsanleitung enthält insbesondere Informationen und Anweisungen für den Verbraucher (m/f) zur Anwendung der Mittel aus den Behältern der Verpackungseinheit in einem Verfahren gemäß dem ersten Erfindungsgegenstand. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der vorliegenden Erfindung ist ein Verfahren zur Farbveränderung menschlicher Haare, wobei ein Mittel des ersten Erfindungsgegenstands auf das Haar aufgetragen wird, für einen Zeitraum von 3 bis 45 Minuten, bevorzugt 5 bis 30 Minuten im Haar belassen wird, und das Haar anschließend mit Wasser und/oder einem handelsüblichen Shampoo gespült wird.

Die Auftragungs- und die Einwirktemperatur der Farbänderungszubereitung beträgt Raumtemperatur bis 45°C. Gegebenenfalls kann die Wirkung der Zubereitung durch externe Wärmezufuhr, wie mittels einer Wärmehaube, verstärkt werden. Die bevorzugte Einwirkdauer der Zubereitung auf die keratinische Faser beträgt 3 bis 45 min, bevorzugt 5 bis 30 min. Nach Beendigung der Einwirkdauer wird das verbliebene Farbveränderungsmittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet. Die Auftragung der Zubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbezubereitung mit einer Applikationshilfe auf die keratinischen Fasern aufzutragen.

In einer Ausführungsform dieses Verfahrens wird das anwendungsbereite Färbemittel durch Vermischen der Färbezubereitung aus Container C1 mit der Entwicklerzubereitung aus Container C2 der Mehrkomponentenverpackungseinheit des zweiten Erfindungsgegenstands hergestellt, auf die keratinischen Fasern aufgetragen, für eine bestimmte Einwirkzeit im Haar belassen und das Haar anschließend mit Wasser und/oder einem handelsüblichen Shampoo ausgespült.

Ein weiterer Gegenstand der Erfindung ist die kosmetische, nicht-therapeutische Verwendung eines Farbveränderungsmittels des ersten Erfindungsgegenstands zur Verbesserung der inneren Haarstruktur bei der oxidativen Färbung menschlicher Haare. Ein weiterer Gegenstand der Erfindung ist die kosmetische, nicht-therapeutische Verwendung eines Farbveränderungsmittels des ersten Erfindungsgegenstands zur Verbesserung des Glanzes der gefärbten keratinischen Fasern. Bezüglich weiterer bevorzugter Ausführungsformen der erfindungsgemäßen Verfahren und Verwendungen gilt mutatis mutandis das zu den erfindungsgemäßen Mitteln Gesagte.

### Beispiele

### 1.1 Herstellung der Färbecreme

Folgende Cremes wurden hergestellt:

| Rohstoffe | E1 | V1 | E2 | E3 | E4 |
|---|---|---|---|---|---|
| p-Toluylendiaminsulfat | -- | -- | 2,54 | 1,06 | -- |
| Resorcin | | -- | 0,90 | 0,33 | -- |
| 3-Aminophenol | -- | -- | 0,30 | 0,14 | 0,44 |
| 5-Amino-2-methylphenol | -- | -- | -- | 0,41 | 0,33 |
| 4,5-Diamino-1-(hydroxyethyl)pyrazolsulfat | -- | -- | -- | 0,70 | 1,35 |
| 2-Amino-4-hydroxyethylaminoanisolsulfat | -- | -- | 0,10 | 0,02 | -- |
| 4-Amino-3-methylphenol | -- | -- | -- | -- | 0,14 |
| Silica, hydrophil | -- | -- | 0,25 | -- | -- |
| Ammonium Carbomer, 1% | 12,0 | 12,0 | 12,0 | 12,0 | 12,0 |
| Lanette E, Pulver | 0,6 | 0,6 | 0,6 | 0,6 | 0,6 |
| Texapon NSF, 27% | 3,5 | 3,5 | 3,5 | 3,5 | 3,5 |
| Kalium Oleat, 12,5% | 2,4 | 2,4 | 2,4 | 2,4 | 2,4 |
| Cutina GMS SE | 1,6 | 1,6 | 1,6 | 1,6 | 1,6 |
| Cutina AGS | 1,6 | 1,6 | 1,6 | 1,6 | 1,6 |
| Eutanol G | 1,6 | 1,6 | 1,6 | 1,6 | 1,6 |
| Cetearyl Alcohol | 9,6 | 9,6 | 9,6 | 9,6 | 9,6 |
| Ceteareth-20 | 2,4 | 2,4 | 2,4 | 2,4 | 2,4 |
| L-Serin | 0,5 | 0,5 | 0,5 | 0,5 | 0,5 |
| Phospholipid EFA | 0,1 | 0,1 | 0,1 | 0,1 | 0,1 |
| Na₄-EDTA, Pulver, 87% | 0,2 | 0,2 | 0,2 | 0,2 | 0,2 |
| Süßorangenöl | 0,18 | -- | 0,18 | 0,18 | 0,18 |
| Aprikosenkernöl | 0,5 | -- | 0,5 | 0,5 | 0,5 |
| Fruitliquid Pomegranate NP | 0,5 | -- | 0,5 | 0,5 | 0,5 |
| Ascorbinsäure | 0,1 | 0,2 | 0,1 | 0,1 | 0,1 |
| Natriumsulfit, wasserfrei, 96% | 0,2 | 0,2 | 0,2 | 0,2 | 0,2 |
| Ammoniak, 25% | 8,0 | 8,0 | 8,0 | 8,0 | 8,0 |
| Parfum | qs | qs | qs | qs | qs |
| Wasser | ad 100 | ad 100 | ad 100 | ad 100 | ad 100 |

- Lanette E, Pulver: INCl-Bezeichnung: Sodium Cetearyl Sulfate (Cognis)
- Texapon NSF, 27%: INCl-Bezeichnung: Sodium Laureth Sulfate (Cognis)
- Cutina GMS SE: INCl-Bezeichnung: Glyceryl Stearate (Cognis)
- Cutina AGS: INCl-Bezeichnung: Glycol Distearate (Cognis)
- Eutanol G: INCl-Bezeichnung: Octyldodecanol (Cognis)
- Phospholipid EFA: INCl-Bezeichnung: Linoleamidopropyl PG-dimonium chloride phosphate (Uniqema)
- Fruitliquid Pomegranate NP: INCl-Bezeichnung: Propylene Glycol, Aqua, Punica Granatum Fruit extract (Crodarom)

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V1 handelt es sich um eine nicht erfindungsgemäße Vergleichsrezeptur. Die Rezepturen E1 bis E4 sind erfindungsgemäße Beispiele.

### 1.2. Vermischen mit der Entwicklerdispersion (EW) und Applikation

Jede Färbecreme wurde im Gewichtsverhältnis 1:1 mit einer wie folgt zusammengesetzten Entwicklerdispersion ausgemischt.

| Rohstoff | Gew.-% |
|---|---|
| Na-benzoat | 0,04 |
| Dipicolinsäure | 0,10 |
| Dinatriumpyrophosphat | 0,19 |
| 1,2-Propandiol | 0,50 |
| HEDP, 60% | 0,25 |
| Paraffinum Liquidum | 0,30 |
| Genamin STAC | 0,20 |
| Cetearyl Alcohol | 3,00 |
| Eumulgin B 2 | 0,70 |
| Wasserstoffperoxid 50 % | 12,2 |
| Kaliumhydroxid, 50 % | 0,19 |
| Wasser | ad 100 |

Genamin STAC Trimethylstearylammonium chlorid (ca. 80% Aktivsubstanz; INCI-Bezeichnung: Steartrimonium Chloride) (Clariant)

Strähnen von natürlich dunklem Haar (natural dark European Alkinco 6634) wurden mit 10 Gew.-% Natrium-Laurylethersulfat-Lösung im Ultraschallbad für 15 min behandelt, anschließend 10 min mit lauwarmen Wasser gespült. Die Strähnen wurden an der Luft getrocknet und für 48 h bei 25°C und 40% relativer Luftfeuchtigkeit gelagert.

Für die Färbung wurde auf den Strähnen von ca. 0,7 g Gewicht die 4-fache Menge der fertigen Anwendungsmischungen appliziert. Nachdem die Strähnen für 30 min bei 32 °C gefärbt wurden, wurden sie für 1 min mit lauwarmem Wasser gespült und an der Luft getrocknet.

### 2 Untersuchung der Schädigung der inneren Haarstruktur mittels DSC-Messungen

### 2.1 DSC-Messungen (Hintergrund)

Mittels DSC-Messungen (Differential Scanning Calorimetry) lassen morphologische Unterschiede in der Faserstruktur identifizieren. Vereinfacht kann das Haar als ein 2-Phasen-Kompositmaterial aus helicalen Faserkristallen in einer amorphen Matrix angesehen werden. Diese Komponenten sind hauptsächlich für mechanische Eigenschaften und daran geknüpfte Erscheinungsformen wie Glanz, Elsatiziät und Geschmeidigkeit verantwortlich. Durch DSC-Messungen werden Schmelzpunkte der Faserstruktur ermittelt, die als Indikator für Schädigungen oder Verbesserungen in sowohl der helicalen Faserkristallen als auch der umgebenden Matrix dienen können. Je kleiner die Kristalle der helicalen Phase sind und desto weniger verlinkt die amorphe Matrix ist, desto niedriger ist die ermittelte Temperatur. Die DSC-Temperatur ist daher ein Maß für die Integrität oder Schädigung einer inneren Haarfaserstruktur.

### 2.2 Durchführung der DSC-Messungen

Die behandelten Strähnen wurden jeweils in ca. 1 mm Stücke geschnitten und in DSC-Probengefäße überführt. Pro behandelter Strähne wurden je 4 Aliquots untersucht. Die Probengefäße wurden je mit 50 µL deionisiertem Wasser aufgefüllt und versiegelt.

Die Messungen wurden an einem DSC-Gerät der Firma Perkin Elmer (DSC 7) durchgeführt. Dabei wurde in einem Temperaturbereich von 110 bis 170°C bei einer Heizrate von 10 K·min⁻¹ gemessen.

### 2.3 Ergebnisse

Folgende DSC-Phasenübergangstemperaturen wurden dabei bestimmt (Tabelle 1):

| Haartyp | DSC-Temperatur [°C] |
|---|---|
| unbehandelt | 151.3 |
| behandelt mit V1 + EW | 150.7 |
| behandelt mit E1 + EW | 151.7 |

Es zeigt sich, dass die mit dem erfindungsgemäßen Mittel (E1 + EW) behandelten Haarsträhnen eine signifikant erhöhte Phasenübergangstemperatur gegenüber den Vergleichssträhnen (V1 + EW) aufweisen. Das bedeutet, dass die mit dem erfindungsgemäßen Mittel behandelten Haare über eine verbesserte, innere Faserstruktur verfügen.

## Patentansprüche

1. Mittel zur Färbung und/oder Aufhellung keratinischer Fasern, insbesondere menschlicher Haare, enthaltend in einem kosmetischen Träger mindestens eine farbverändernde Komponente, **dadurch gekennzeichnet, dass** das Mittel zusätzlich eine Wirkstoffkombination aus
(a) mindestens einem wässrig-alkoholischen Extrakt aus *Punica granatum L.,*
(b) mindestens einem Fettsäuretriglycerid, welches einen durchschnittlichen Anteil an Estern von ungesättigten Fettsäuren von wenigstens 80 %, bezogen auf die Gesamtmenge an Fettsäureestern des Triglycerids, aufweist,
(c) und mindestens einem ätherischen Öl, enthaltend einen Anteil an Terpenen von mindestens 95 Gew.-%, bezogen auf das Gesamtgewicht des pflanzlichen Öls, enthält.

2. Mittel nach Anspruch 1, **dadurch gekennzeichnet, dass** die farbverändernde Komponente ausgewählt ist aus mindestens einem Oxidationsfarbstoffvorprodukt vom Typ der Entwicklerkomponenten und gegebenenfalls zusätzlich mindestens einer Kupplerkomponente und/oder aus mindestens einem direktziehenden Farbstoff.

3. Mittel nach einem der Ansprüche 1 oder 2, **dadurch gekennzeichnet, dass** das Fettsäuretriglycerid (b) einen durchschnittlichen Anteil an Estern von Linolsäure von wenigstens 25 %, bezogen auf die Gesamtmenge an Fettsäureestern des Triglycerids, aufweist.

4. Mittel nach einem der Ansprüche 1 bis 3, **dadurch gekennzeichnet, dass** das Mittel als Fettsäuretriglycerid (b) das Kernöl von *Prunus Armeniaca* enthält.

5. Mittel nach einem der Ansprüche 1 bis 4, **dadurch gekennzeichnet, dass** das ätherische Öl (c) einen durchschnittlichen Anteil an Limonen von wenigstens 90 Gew.-%, bezogen auf das Gesamtgewicht des ätherischen Öls (c), aufweist.

6. Mittel nach einem der Ansprüche 1 bis 5, **dadurch gekennzeichnet, dass** das Mittel als ätherisches Öl (c) ein Öl aus Schalen von *Citrus sinensis* enthält.

7. Mittel nach einem der Ansprüche 1 bis 6, **dadurch gekennzeichnet, dass** das Mittel das Fettsäuretriglycerid (b) in einem Gewichtsanteil von 0,001 bis 5 Gew.-%, bevorzugt von 0,01 bis 3 Gew.-%, und insbesondere von 0,1 bis 1,5 Gew.-% und das ätherische Öl (c) in einem Gewichtsanteil von 0,001 bis 2 Gew.-%, bevorzugt von 0,01 bis 1,5 Gew.-%, und insbesondere von 0,05 bis 1,0 Gew.-% enthält, wobei sich die Gewichtsanteile jeweils auf das Gesamtgewicht des anwendungsbereiten Mittels beziehen.

8. Mittel nach einem der Ansprüche 1 bis 7, **dadurch gekennzeichnet, dass** das anwendungsbereite Mittel mindestens ein Oxidationsfarbstoffvorprodukt vom Typ der Entwicklerkomponenten, mindestens ein Oxidationsfarbstoffvorprodukt vom Typ der Kupplerkomponente und zusätzlich mindestens ein chemisches Oxidationsmittel, insbesondere Wasserstoffperoxid, enthält.

9. Kosmetische, nicht-therapeutische Verwendung eines Mittels nach einem der Ansprüche 1 bis 8 zur Verbesserung der inneren Haarstruktur bei der oxidativen Färbung menschlicher Haare.

10. Mehrkomponentenverpackungseinheit (Kit-of-Parts), umfassend mindestens zwei voneinander getrennt konfektionierte Verpackungseinheiten (Container), **dadurch gekennzeichnet,**
**dass** ein Container C1 in einem kosmetischen Träger mindestens ein Oxidationsfarbstoffvorprodukt vom Typ der Entwicklerkomponenten, gegebenenfalls zusätzlich mindestens einer Kupplerkomponente, sowie mindestens eine Wirkstoffkombination aus
(a) mindestens einem wässrig-alkoholischen Extrakt aus *Punica granatum L.,*
(b) mindestens einem Fettsäuretriglycerid, welches einen durchschnittlichen Anteil an Estern von ungesättigten Fettsäuren von wenigstens 80 %, bezogen auf die Gesamtmenge an Fettsäureestern des Triglycerids, aufweist,
(c) und mindestens ein ätherisches Öl, enthaltend einen Anteil an Terpenen von mindestens 95 Gew.-%, bezogen auf das Gesamtgewicht des pflanzlichen Öls, enthält, und
**dass** ein weiterer Container C2 in einem kosmetischen Träger mindestens ein chemisches Oxidationsmittel, insbesondere Wasserstoffperoxid, enthält.
